(19) Europäisches Patentamt
European Patent Office
Office européen des brevets

(11) EP 1 001 950 B1

(12) **EUROPEAN PATENT SPECIFICATION**

(45) Date of publication and mention of the grant of the patent:
**04.12.2002 Bulletin 2002/49**

(21) Application number: **98935122.6**

(22) Date of filing: **16.07.1998**

(51) Int Cl.[7]: **C07D 251/70**, C08F 20/68

(86) International application number:
**PCT/GB98/02104**

(87) International publication number:
**WO 99/005127 (04.02.1999 Gazette 1999/05)**

(54) **FLUORINATED TRIAZINE MONOMERS**

FLUORIERTE TRIAZIN MONOMERE

MONOMERES DE TRIAZINE FLUORES

(84) Designated Contracting States:
**AT BE CH DE DK ES FI FR IE IT LI LU NL PT SE**

(30) Priority: **26.07.1997 GB 9715709**

(43) Date of publication of application:
**24.05.2000 Bulletin 2000/21**

(73) Proprietor: **The Secretary of State for Defence
Salisbury, Wiltshire SP4 0JQ (GB)**

(72) Inventors:
- **WILLIS, Colin, Robert
Salisbury, Wiltshire SP4 0JQ (GB)**
- **BREWER, Stuart, Anson
Salisbury, Wiltshire SP4 0JQ (GB)**
- **JONES, Brian, George
Salisbury, Wiltshire SP4 0JQ (GB)**

(74) Representative: **Skelton, Stephen Richard et al
D/IPR Formalities Section,
Poplar 2,
MOD Abbey Wood, nr. 2218
Bristol BS34 8JH (GB)**

(56) References cited:
**EP-A- 0 366 884**
**WO-A-97/13024**
**GB-A- 1 102 903**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

EP 1 001 950 B1

## Description

The present invention relates to novel monomeric compounds which can be used in the production of polymers which have a high degree of oil and water-repellency and which may be fixed to substrates such as clothing, to processes for their preparation and to polymers produced therefrom.

Oil- and water- repellent treatments are in widespread use, in particular for outdoor clothing applications, sportswear, leisurewear and in military applications. These treatments generally require the incorporation of a fluoropolymer into or more particularly, fixed onto the surface of the clothing fabric. The degree of oil and water repellency is a function of the number of fluorocarbon groups or moieties that can be fitted into the available space. The greater the concentration of such moieties, the greater the repellency of the finish.

In addition however, the polymeric compounds must be able to form durable bonds with the substrate. Oil- and water-repellent textile treatments are generally based on fluoropolymers that are applied to fabric in the form of an aqueous emulsion. The fabric remains breathable and permeable to air since the treatment simply coats the fibres with a very thin, liquid-repellent film. In order to make these finishes durable, they are sometimes co-applied with cross-linking resins that bind the fluoropolymer treatment to fibres. Whilst good levels of durability towards laundering and dry-cleaning can be achieved in this way, the cross-linking resins can seriously damage cellulosic fibres and reduce the mechanical strength of the material.

WO 97/13024 discloses a group of fibre reactive polymers, which include a functional group such as a triazine group, which binds the polymer to the material substrate.

British patent No 1,102,903 describes certain fluoro alkyl containing compounds which are used in water- and oil-repellent compositions.

The applicants have produced certain novel monomers, which give rise to polymers which have a high number of fluorocarbon substituents per monomer unit.

The present invention provides a compound of formula (I)

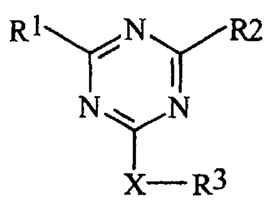

(I)

wherein $R^1$ and $R^2$ are independently selected from $NR^5(CH_2)_nC_mF_{2m+1}$, $O(CH_2)_nC_mF_{2m+1}$, $S(CH_2)_nC_mF_{2m+1}$, $NR^5S(O)_2(CH_2)_pC_mF_{2m+1}$ or $CR^5[CO_2(CH_2)_nC_mF_{2m+1}]_2$, where $R^5$ is hydrogen or a straight or branched chain alkyl or cycloalkyl group having from 1-12 carbon atoms, n and m are independently an integer of 1-12, and p is 0 or an integer of from 1-12; $R^3$ comprises an alkene or alkyne group which may be polymerised, and X is O, S or $NR^4$ where $R^4$ is hydrogen or a straight or branched chain alkyl or cycloalkyl group having from 1-12 carbon atoms.

As used herein, the term "alkyl" refers to straight or branched chain alkyl or cycloalkyl groups having from 1 to 12 and preferably from 1 to 6 carbon atoms. The term "saturated" refers to groups which do not contain carbon-carbon double bonds. Conversely the term "unsaturated" refers to groups which include carbon-carbon double bonds.

Conveniently $R^1$ and $R^2$ are the same. They are preferably selected from $O(CH_2)_nC_mF_{2m+1}$ or $NR^5S(O)_2(CH_2)_pC_mF_{2m+1}$. Suitably $R^5$ is methyl, ethyl or n-propyl, in particular ethyl. Preferred integers for n and p are from 1-3, suitably 2, whilst preferred integers for m are from 6 to 10, most preferably 8.

Suitable polymerisable groups $R^3$ are alkenes or alkynes which may also include a functional group such as an acyloxy group. Particularly preferred groups for $R^3$ are groups of formula $(CH_2)_qOC(O)\ C(R^6)=CR^7R^8$ where q is an integer of from 1 to 12, suitably from 1 to 4 and especially 2, and $R^6$, $R^7$ and $R^8$ are independently selected from hydrogen or $C_{1-4}$ alkyl. Preferably $R^6$, $R^7$ and $R^8$ are all hydrogen.

Compounds of formula (I) may be prepared by reacting a compound of formula (II)

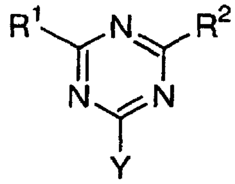

(II)

where $R^1$ and $R^2$ are as defined with relation to formula (I) and Y is a leaving group,
with a compound of formula (III)

$$R^a - X - R^{3'} \qquad (III)$$

where X is defined in relation to formula (I), $R^{3'}$ is a group $R^3$ as defined in relation to formula (I) or a precursor group which may be reacted to form a group $R^3$ and $R^a$ is hydrogen or $C_{1-3}$ alkyl; and thereafter if necessary converting a precursor group $R^{3'}$ to a group $R^3$.

Preferably $R^a$ is hydrogen or methyl.

Suitable leaving groups for Y include halogen such as fluorine and chlorine, in particular chlorine, or amine leaving groups such as substituted pyridinyl groups for instance nicotinate group or collidinyl group.

The reaction is suitably effected in an organic solvent such as tetrahydrofuran (THF), acetone, toluene or chloroform. It may be effected at temperatures of from 0 to 200°C, suitably from 25 to 150°C, depending upon the precise nature of the reactants and solvents involved. Conveniently the reaction may be effected at room temperature or under reflux conditions.

Preferably the reaction is effected under basic conditions. Weak bases may suffice, and in some instances, the compound of formula (III) may itself act as an acid scavenger and so the use of an excess, particularly a 2 molar excess of the compound of formula (III) will ensure that that the reaction proceeds effectively.

Suitable groups $R^{3'}$ which are precursors to $R^3$ would be apparent to the skilled person. For example, where $R^3$ is a group of formula $(CH_2)_qOC(O)\ C(R^6)=CR^7R^8$, a suitable precursor group $R^{3'}$ would be $(CH_2)_qOH$ which can be readily converted to $R^3$ by reaction with a suitable acid halide for example an acid chloride of formula $ClC(O)C(R^6)=CR^7R^8$ in the presence of a base, for example pyridine or a pyridine derivative such as collidine. This reaction is suitably effected in an organic solvent such as toluene at elevated temperatures, conveniently at the reflux temperature of the solvent.

Certain compounds of formula (II) are known (see for example British Patent No. 1,102,903). These compounds can be prepared by reacting a compound of formula (IV)

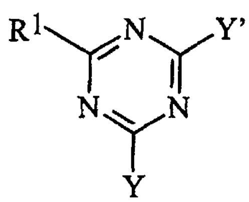

(IV)

where $R^1$ is as defined in relation to formula (I), Y is as defined in relation to formula (II) and Y' is a leaving group, with a compound of formula (V)

$$R^2H \qquad (V)$$

where $R^2$ is as defined in relation to formula (I), in the presence of a base.

Suitable bases are those which react with a compound of formula (V) so as to produce a nucleophilic moiety

of formula (V')

$$(R^2)^- \qquad (V')$$

Thus the selection of suitable bases will depend upon the precise nature of the group $R^2$ and will be readily understood or determinable by the skilled person. For example, where $R^2$ is a group $O(CH_2)_nC_mF_{2m+1}$, strong bases such as alkali metal hydroxides, in particular lithium hydroxide, may be used. Alternatively, where $R^2$ is a group $NR^5S(O)_2(CH_2)_pC_mF_{2m+1}$, stronger bases such as alkali metal alkoxides, in particular sodium or potassium methoxide or ethoxide may be used.

Compounds of formula (IV) are suitably prepared by reacting a compound of formula (VI)

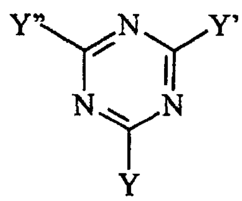

(VI)

wherein Y, Y'and Y" are the same or different leaving groups, with a compound of formula (VII)

$$R^1H \qquad (VII)$$

where $R^1$ is as defined in relation to formula (I), in the presence of a base.

Reaction conditions will be generally similar to those described above in relation to the reaction between compounds of formula (IV) and formula (V).

Where compounds of formula (V) and formula (VII) are the same, compounds of formula (II) may be prepared directly in one pot. If necessary, the reaction can be controlled in a stepwise manner in order to maximise yield of the target compound by controlling the reaction temperature. For example, where $R^1$ and $R^2$ are groups of formula $NR^5S(O)_2(CH_2)_nC_mF_{2m+1}$, the compound of formula (IV) may be prepared at depressed temperatures, for example at about -78°C. Allowing the reaction mixture to warm up to approximately 0°C will produce a compound of formula (II) after suitable work-up.

Compounds of formula (III), (V), (VI) and (VII) are either known compounds or they can be prepared from known compounds using conventional methods. A preferred compound of formula (VI) is cyanuric chloride.

Compounds of formula (I) may be polymerised or copolymerised using conventional technology, e.g. emulsion polymerisation.

Polymers or co-polymers including repeat units of formula (VIII)

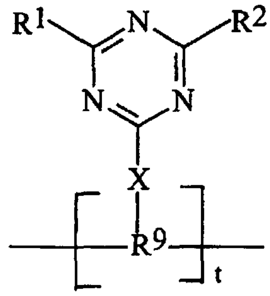

(VIII)

where $R^1$, $R^2$ and X are as defined in relation to formula (I), t is an integer in excess of 5, and $R^9$ is a saturated derivative of $R^3$ as defined in relation to formula (I) form a preferred embodiment of the invention. In particular $XR^9$ will be a moiety of formula (IX)

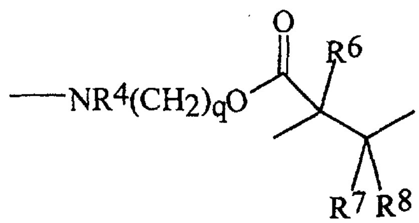

(IX)

Suitably the monomers of the invention are copolymerised with a monomer which comprises a fibre reactive moiety for example as described in WO 97/13024.

The present invention further provides a substrate which is coated with a polymeric compound which has been derived from a compound of formula (I), particularly those polymeric compounds which comprise a polymer or copolymer including repeating units of formula (VIII), preferably wherein $XR^9$ is a moiety of formula (IX). The substrate is preferably a fabric.

The invention will now be particularly described by way of example.

**Example 1**

**Step 1**

**Synthesis of 2-chloro-4,6-bis(N-ethylperfluorooctylsulphonamido)-1,3,5-triazine**

Metallic sodium (4.08g, 177mmols) was reacted with methanol (150mls). N-Ethyl perfluorooctyl sulphonamide (93.28g, 177mmols) was added, and the resulting solution was stirred for 30 minutes. The methanol was removed at the pump (a vacuum pump was required to remove the final traces of solvent). The resulting sticky solid was dissolved in acetone (300mls) and cooled to -65°C under argon. Recrystalised cyanuric chloride (16.33g, 88.5mmols) dissolved in acetone (100mls) was added to the reaction mixture dropwise such that the temperature did not rise above -50°C (~1hour). After the addition, the reaction mixture was allowed to slowly warm to room temperature (1 hour) and then stirred for a further 3 hours. The precipated solid was removed by filtration and dried under vacuum.

Purification by soxhlet extraction with acetone afforded 61g (56.9%) of a fine white powder.

$^1$H NMR ($CDCl_3$) δ (ppm) 4.20 (2H, q, $^3J_{H-H}$ 6.8 Hz, $\underline{CH_2}CH_3$), 1.40 (3H, t, $^3J_{H-H}$ 6.8 Hz, $CH_2\underline{CH_3}$).

$^{13}$C{$^1$H} NMR ($CDCl_3$) δ (ppm) 171.3, 165.0 (triazine), 46.3 ($\underline{C}H_2CH_3$), 14.5 ($CH_2\underline{C}H_3$).

**Step 2**

**Synthesis of 2-[{4,6-bis(N-ethylperfluorooctylsulphonamido)- 1,3,5-triazin-2-yl}-amino] ethanol**

A THF solution (85mls) of 2-chloro-4,6-bis(N-ethylperfluorooctylsulphonamido)- 1,3,5-triazine (15g, 12.9mmols) and ethanolamine (1.6g, 26.2mmols) were heated under reflux for 1 hour. The hot solution/suspension was filtered and the product was allowed to crystalise overnight to afford 12.8g (83%) of product.
$^1$H NMR ($d_6$ acetone) δ (ppm) 4.85 (4H, m, $N\underline{C}H_2CH_3$), 4.38 (2H, t, $^3J_{H-H}$ 5 Hz, $CH_2O$), 4.22 (2H, dt, 5, 5 Hz, $OCH_2\underline{C}H_2N$), 2.05 (6H, m $NCH_2\underline{C}H_3$).

**Step 3**

**Synthesis of 2-[{4,6-bis(N-ethylperfluorooctylaulphonamido)-1,3,5-triazin-2-yl }-amino]ethyl propenoate**

2- [{4,6-bis(N-ethylperfluorooctylsulphonamido)-1,3,5-triazin-2-yl}amino]ethanol (11.58, 9.7mmols) and acryloyl chloride (1.32g, 14.6mmols) were dissolved in hot toluene (80mls). Collidine (1.77g, 14.6mmols) was added as a toluene solution (10mls) down the reflux condenser. The resulting reaction mixture was heated under reflux for 2 hours and then filtered hot. Toluene was removed at the pump and the resulting solid dissolved in diethyl ether (400mls). The etheral solution was washed with 1M HCl (2x50mls) distilled water (2x40mls) and then dried over sodium sulphate. Filtration and evaporation of the solvent at the pump afforded 8.8g (73%) of product.
$^1$H NMR ($CDCl_3$) δ (ppm) 6.42 (1H, dd, $^3J_{H-H}$ 17.3, 1.3 Hz, $CH{=}\underline{CH_2}_{\ trans}$), 6.11 (1H, dd, $^3J_{H-H}$ 17.3, 10.5 Hz, $\underline{CH}{=}CH_2$), 5.88 (2H, m, NH, $CH{=}\underline{CH_2}_{\ cis}$), 4.32 (2H, t, $^3J_{H-H}$ 5.3 Hz, $CH_2O$), 4.13 (4H, m, $N\underline{CH_2}CH_3$), 3.72 (2H, m, $OCH_2\underline{CH_2}N$), 1.36 (6H, m, $NCH_2\underline{CH_3}$).
$^{13}C\{^1H\}$ NMR ($CDCl_3$) δ (ppm) 166.0, 165.6, 164.6, 164.3, (triazine/C=O), 131.5 (C=C), 127.8 (C=C), 62.5 ($\underline{C}H_2O$), 45.4 ($CH_3\underline{C}H_2N$), 40.5 ($\underline{C}H_2N$), 14.9 ($\underline{C}H_3CH_2$).

**Example 2**

**Synthesis of 2-[N-methyl-N-{ 4,6-bis(N-ethylperfluorooctylsulphonamido)-1,3,5-triazin-2-yl }-amino]ethyl propenoate**

2-Chloro-4,6-bis(N-ethylperfluorooctylsulphonamido)-1,3,5-triazine (20g, 17.2mmols) was held as a solution/suspension in chloroform (150mls). N,N-Dimethylethylamino acrylate (2.45g, 17.2mmols) was added dropwise, over a period of 30 minutes, as a chloroform solution (50mls). The reaction mixture was stirred for 3 hours at room temperature. The chloroform solution was filtered through Celite®, concentrated (to a volume of approximately 30mls) and then passed through a short path column of silica. Product was eluted with chloroform.
Evaporation of the solvent afforded 19g (88%) of a sticky oil that crystalised with time (2 days).
$^1$H NMR ($CDCl_3$) δ (ppm) 6.37 (1H, dd, $^3J_{H-H}$ 17.3, 1.5 Hz, $CH{=}\underline{CH_2}_{\ trans}$), 6.09 (1H, dd, $^3J_{H-H}$ 17.3, 10.5 Hz, $CH{=}CH_2$), 5.83 (1H, dd, $^3J_{H-H}$ 10.5, 1.5 Hz, $CH{=}\underline{CH_2}_{\ cis}$), 4.36 (2H, t, $^3J_{H-H}$ 5.6 Hz, $CH_2O$), 4.14 (4H, m, $N\underline{C}H_2CH_3$), 3.86 (2H, t, $^3J_{H-H}$ 5.6 Hz, $OCH_2\underline{C}H_2N$), 3.20 (3H, s, $CH_3N$), 1.38 (6H, m, $NCH_2\underline{C}H_3$).
$^{13}C\{^1H\}$ NMR ($CDCl_3$) δ (ppm) 165.8, 164.5, 164.2, 164.0, (triazine/C=O), 131.3 (C=C), 127.9 (C=C), 61.7 ($\underline{C}H_2O$), 48.3 ($\underline{C}H_2N$), 45.5 ($\underline{C}H_2N$), 36.3 ($CH_3N$), 15.0 ($\underline{C}H_3CH_2$).

**Example 3**

**Step 1**

**Synthesis of 2,4-Bis(1H,1H,2H,2H-perfluorooctoxy)-6-chloro-1,3,5-triazine**

Lithium hydroxide (0.49g, 11.7mmols) and 1H,1H,2H,2H perfluorooctanol (5.4g 11.7mmols) were held as a solution/suspension in tetrahydrofuran (25mls). Cyanuric chloride (1.08g, 5.8mmols) and distilled water (1ml) were added and the reaction mixture was stirred at room temperature overnight. The resulting soluton/suspension was precipitated into distilled water (200mls) and extracted with diethyl ether (2x200mls). The organic extract was dried over sodium sulphate, filtered and the diethyl ether was removed at the pump. The resulting white solid was recrystalised form diethyl ether (50mls), to afford 3.3g (54%) of product.
$^1$H NMR ($CDCl_3$) δ (ppm) 4.75 (2H, t, $^3J_{H-H}$ 6.6 Hz, $O\underline{CH_2}CH_2$), 2.63 (2H, tt, $^3J_{H-F}$ 18.1 Hz, $^3J_{H-H}$ 6.6 Hz $OCH_2\underline{CH_2}$).
$^{13}C\{^1H\}$ NMR ($CDCl_3$) δ (ppm) 173.2, 171.7 (triazine), 61.1 ($O\underline{C}H_2CH_2$), 30.5 (t, $^2J_{C-F}$ 22.0 Hz $OCH_2\underline{CH_2}CF_2$).

**Step 2**

**Synthesis of 2-[N-methyl-N-{ 4,6-bis(1H,1H,2H,2H-perfluorooctoxy)- 1,3,5-triazin-2-yl } amino]ethyl propenoate**

2,4-Bis(1H,1H,2H,2H-perfluorooctoxy)-6-chloro-1,3,5-triazine (0.5g, 0.48mmols) was held as a solution/suspension in chloroform (10mls). N,N-Dimethylethyl-amino acrylate (0.076g, 0.53mmols) was added dropwise as a neat liquid at room temperature and the reaction mixture was stirred for 2 hours. The chloroform solution was extracted with 2M HCl (2x10mls), distilled water (2x10mls), dried over sodium sulphate and filtered. Evaporation of the solvent afforded 0.48g (90%) of product as a waxy solid.

$^1H$ NMR ($CDCl_3$) $\delta$ (ppm) 6.30 (1H, d, $^3J_{H-H}$ 17.2 Hz, $CH=\underline{CH_2}_{trans}$), 6.00 (1H, dd, $^3J_{H-H}$ 17.3, 10.4 Hz, $\underline{CH}=CH_2$), 5.75 (1H, d, $^3J_{H-H}$ 10.4 Hz, $CH=\underline{CH_2}_{cis}$), 4.57 (4H, m, $CF_2CH_2\underline{CH_2}O$), 4.31 (2H, t, 5.5 Hz, $O\underline{CH_2}CH_2N$), 3.84 (2H, t, $^3J_{H-H}$ 5.5 Hz, $OCH_2\underline{CH_2}N$), 3.14 (3H, s, $CH_3N$), 2.56 (4H, m, $CF_2\underline{CH_2}CH_2O$).

$^{13}C\{^1H\}$ NMR ($CDCl_3$) $\delta$ (ppm) 171.3, 171.1, 167.3, 165.8, (triazine/C=O), 131.1 (C=C), 128.0 (C=C), 61.8 ($\underline{C}H_2O$), 59.2 ($\underline{C}H_2N$), 48.0 ($\underline{C}H_2N$), 36.1 ($CH_3N$), 30.2 (t, 22 Hz, $\underline{C}H_2CF_2$).

## Claims

1. A compound of formula (I)

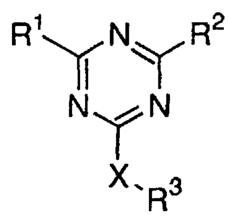

(I)

wherein $R^1$ and $R^2$ are independently selected from $NR^5(CH_2)_nC_mF_{2m+1}$, $O(CH_2)_nC_mF_{2m+1}$, $S(CH_2)_nC_mF_{2m+1}$, $NR^5S(O)_2(CH_2)_pC_mF_{2m+1}$ or $CR^5[CO_2(CH_2)_nC_mF_{2m+1}]_2$, where $R^5$ is hydrogen or a straight or branched chain alkyl or cycloalkyl group having from 1-12 carbon atoms, n and m are independently an integer of 1-12, and p is 0 or an integer of from 1-12; $R^3$ comprises an alkene or alkyne group which may be polymerised, and X is O, S or $NR^4$ where $R^4$ is hydrogen or a straight or branched chain alkyl or cycloalkyl group having from 1-12 carbon atoms.

2. A compound according to claim 1 wherein $R^1$ and $R^2$ are the same.

3. A compound according to either of claims 1 and 2 wherein $R^1$ and $R^2$ are selected from $O(CH_2)_nC_mF_{2m+1}$ or $NR^5S(O)_2(CH_2)_pC_mF_{2m+1}$ where $R^5$, p, m and n are as defined in claim 1.

4. A compound according to any preceding claim wherein n and p are independently an integer of from 1 to 3 and m is an integer of from 6 to 10.

5. A compound according to any one of the preceding claims wherein $R^3$ is a group of formula $-(CH_2)_qOC(O)C(R^6)=CR^7R^8$ where q is an integer of from 1 to 12 and $R^6$, $R^7$ and $R^8$ are independently selected from hydrogen or a $C_{1-4}$ alkyl group.

6. A compound according to claim 5 wherein $R^6$, $R^7$ and $R^8$ are all hydrogen.

7. A method for preparing a compound of formula (I) as defined in claim 1 which method comprises reacting a compound of formula (II)

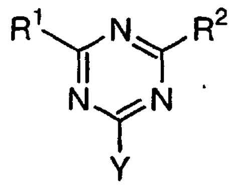

(II)

where $R^1$ and $R^2$ are as defined in claim 1 and Y is a leaving group, with a compound of formula (III)

$$R^a\text{-}X\text{-}R^{3'} \qquad \text{(III)}$$

where X is as defined in claim 1, and $R^{3'}$ is a group $R^3$ as defined in claim 1 or a precursor group which may be reacted to form a group $R^3$ and $R^a$ is hydrogen or a $C_{1-3}$ alkyl group; and thereafter if necessary converting a group $R^{3'}$ to a group $R^3$.

**8.** A method according to claim 7 wherein $R^{3'}$ is a group of formula $(CH_2)_qOH$ and this is subsequently converted to $R^3$ by reaction with an acid halide of formula $ZC(O)C(R^6)=CR^7R^8$ where Z is a halogen and $R^6$, $R^7$ and $R^8$ are as defined in claim 5, in the presence of a base.

**9.** A method according to either of claims 7 and 8 wherein the compound of formula (II) is prepared by reacting a compound of formula (IV)

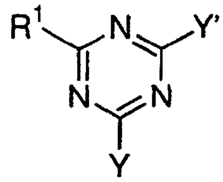

(IV)

where $R^1$ is defined in relation to formula (I), Y is as defined in relation to formula (II) and Y' is a leaving group, with a compound of formula (V)

$$R^2H \qquad \text{(V)}$$

where $R^2$ is as defined in relation to formula (I), in the presence of a base.

**10.** A method according to claim 9 wherein the compound of formula (IV) is prepared by reacting a compound of formula (VI)

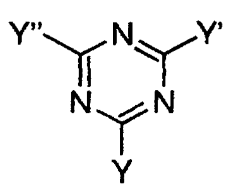

(VI)

wherein Y, Y' and Y" are the same or different leaving groups, with a compound of formula (VII)

$$R^1H \qquad \text{(VII)}$$

where $R^1$ is as defined in claim 1, in the presence of a base.

**11.** A method according to claim 10 wherein the compound of formula (IV) is converted to a compound of formula (II) in situ.

**12.** A polymeric compound which has been derived from a compound of formula (I) of claim 1.

**13.** A polymeric compound according to claim 12, which comprises a polymer or co-polymer including repeating units of formula (VIII)

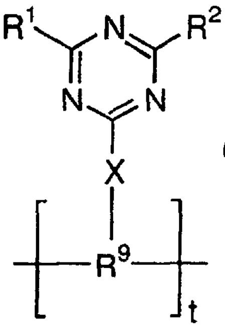

(VIII)

where $R^1$, $R^2$ and X are as defined in relation to formula (I), t is an integer in excess of 5, and $R^9$ is a saturated derivative of $R^3$ as defined in relation to formula (I).

**14.** A polymeric compound according to claim 13 wherein $XR^9$ is a moiety of formula (IX)

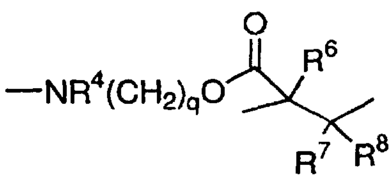

(IX)

where q, $R^6$, $R^7$ and $R^8$ are as defined in claim 5.

**15.** A substrate which is coated with a polymeric compound according to any one of claims 12 to 14.

**16.** A substrate according to claim 15 which is a fabric.

**Patentansprüche**

**1.** Verbindung der Formel (I)

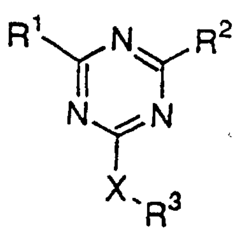

(I),

worin die Gruppen $R^1$ und $R^2$ unabhängig voneinander unter $NR^5(CH_2)_nC_mF_{2m+1}$, $O(CH_2)_nC_mF_{2m+1}$, $S(CH_2)_nC_mF_{2m+1}$, $NR^5S(O)_2(CH_2)_pC_mF_{2m+1}$ und $CR^5[CO_2(CH_2)_nC_mF_{2m+1}]_2$ ausgewählt sind, wobei $R^5$ Wasserstoff

oder eine geradkettige oder verzweigte Alkyl- oder Cycloalkylgruppe mit 1 bis 12 Kohlenstoffatomen bedeutet, n und m unabhängig voneinander eine ganze Zahl von 1 bis 12 bedeuten und p 0 oder eine ganze Zahl von 1 bis 12 ist; $R^3$ eine Alken- oder Alkingruppe enthält, die polymerisiert werden kann, und X O, S oder $NR^4$ bedeutet, wobei $R^4$ Wasserstoff oder eine geradkettige oder verzweigte Alkyl- oder Cycloalkylgruppe mit 1 bis 12 Kohlenstoffatomen ist.

**2.** Verbindung nach Anspruch 1, wobei $R^1$ und $R^2$ identisch sind.

**3.** Verbindung nach Anspruch 1 oder 2, wobei $R^1$ und $R^2$ unter $O(CH_2)_nC_mF_{2m+1}$ oder $NR^5S(O)_2(CH_2)_pC_mF_{2m+1}$ ausgewählt sind, wobei $R^5$, p, m und n die in Anspruch 1 angegebenen Bedeutungen aufweisen.

**4.** Verbindung nach einem der vorhergehenden Ansprüche, wobei n und p unabhängig voneinander eine ganze Zahl von 1 bis 3 bedeuten und m eine ganze Zahl von 6 bis 10 ist.

**5.** Verbindung nach einem der vorhergehenden Ansprüche, wobei $R^3$ eine Gruppe der Formel $-(CH_2)_qOC(O)C(R^6)=CR^7R^8$ ist, wobei q eine ganze Zahl von 1 bis 12 bedeutet und die Gruppen $R^6$, $R^7$ und $R^8$ unabhängig voneinander unter Wasserstoff oder $C_{1-4}$-Alkyl ausgewählt sind.

**6.** Verbindung nach Anspruch 5, wobei $R^6$, $R^7$ und $R^8$ gleichzeitig Wasserstoff bedeuten.

**7.** Verfahren zur Herstellung einer Verbindung der Formel (I) nach Anspruch 1, wobei das Verfahren umfaßt, eine Verbindung der Formel (II)

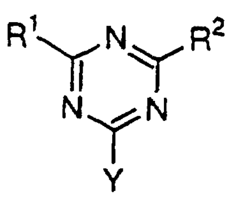

(II),

worin $R^1$ und $R^2$ die in Anspruch 1 angegebenen Bedeutungen aufweisen und Y eine austretende Gruppe ist, mit einer Verbindung der Formel (III) umzusetzen:

$$R^a - X - R^{3'} \qquad (III),$$

worin X die in Anspruch 1 angegebenen Bedeutungen aufweist und $R^{3'}$ eine Gruppe $R^3$ nach der Definition des Anspruchs 1 oder eine Precursorgruppe ist, die zur Bildung einer Gruppe $R^3$ umgesetzt werden kann, und $R^a$ Wasserstoff oder eine $C_{1-3}$-Alkylgruppe bedeutet; und danach erforderlichenfalls die Gruppe $R^{3'}$ in eine Gruppe $R^3$ umzuformen.

**8.** Verfahren nach Anspruch 7, wobei $R^{3'}$ eine Gruppe der Formel $(CH_2)_qOH$ ist, die im Anschluß durch Umsetzung mit einem Säurehalogenid der Formel $ZC(O)C(R^6)=CR^7R^8$, worin Z Halogen bedeutet und $R^6$, $R^7$ und $R^8$ die in Anspruch 5 angegebenen Bedeutungen aufweisen, in Gegenwart einer Base in eine Gruppe $R^3$ umgewandelt wird.

**9.** Verfahren nach Anspruch 7 und 8, wobei die Verbindung der Formel (II) hergestellt wird, indem eine Verbindung der Formel (IV)

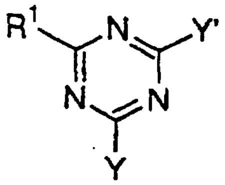

(IV),

worin $R^1$ die für Formel (I) angegebene Definition hat, Y die für Formel (II) angegebene Definition hat und Y' eine austretende Gruppe ist, mit einer Verbindung der Formel (V)

$$R^2H \quad (V),$$

worin $R^2$ die für Formel (I) angegebene Definition hat, in Gegenwart einer Base umgesetzt wird.

**10.** Verfahren nach Anspruch 9, wobei die Verbindung der Formel (IV) hergestellt wird, indem eine Verbindung der Formel (VI)

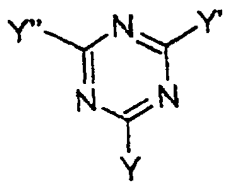

(VI),

worin Y, Y' und Y" identische oder unterschiedliche austretende Gruppen bedeuten, mit einer Verbindung der Formel (VII)

$$R^1H \quad (VII),$$

worin $R^1$ die für Anspruch 1 angegebenen Bedeutungen aufweist, in Gegenwart einer Base umgesetzt wird.

**11.** Verfahren nach Anspruch 10, wobei die Verbindung der Formel (IV) in situ in eine Verbindung der Formel (II) umgewandelt wird.

**12.** Polymere Verbindung, die aus einer Verbindung der Formel (I) nach Anspruch 1 abgeleitet ist.

**13.** Polymere Verbindung nach Anspruch 12, die ein Polymer oder Copolymer mit wiederkehrenden Einheiten der folgenden Formel (VIII) umfaßt:

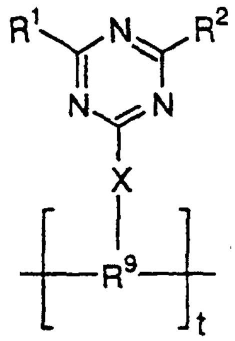

(VIII),

worin die Gruppen $R^1$, $R^2$ und X die für Formel (I) angegebenen Bedeutungen aufweisen, t eine ganze Zahl über 5 ist und $R^9$ ein gesättigtes Derivat von $R^3$ gemäß der für Formel (I) angegebenen Definition ist.

**14.** Polymere Verbindung nach Anspruch 13, wobei $XR^9$ eine Einheit der Formel (IX) ist

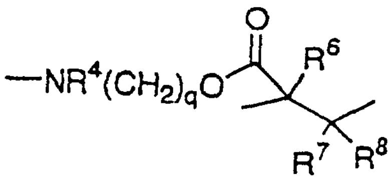

(IX),

worin q, $R^6$, $R^7$ und $R^8$ die in Anspruch 5 angegebenen Bedeutungen aufweisen.

**15.** Substrat, das mit einer polymeren Verbindung nach einem der Ansprüche 12 bis 14 beschichtet ist.

**16.** Substrat nach Anspruch 15, wobei es sich um ein Gewebe handelt.

**Revendications**

**1.** Composé de formule (I)

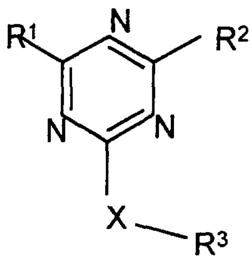

(I)

où $R^1$ et $R^2$ sont choisis indépendamment parmi $NR^5(CH_2)_nC_mF_{2m+1}$, $O(CH_2)_nC_mF_{2m+1}$, $S(CH_2)_nC_mF_{2m+1}$, $NR^5S(O)_2(CH_2)_pC_mF_{2m+1}$ ou $CR^5[CO_2(CH_2)_nC_mF_{2m+1}]_2$ où $R^5$ est u n hydrogène ou une chaîne alkyle linéaire ou ramifiée ou un groupe cycloalkyle ayant de 1 à 12 atomes de carbone, n et m sont indépendamment des entiers de 1 à 12 et p est 0 ou un entier de 1 à 12 ; $R^3$ comprend un groupe alcène ou alcyne qui peut être polymérisé et X est O, S, ou $NR^4$ où $R^4$est un hydrogène ou une chaîne alkyle linéaire ramifiée ou un groupe cycloalkyle ayant de 1 à 12 atomes de carbone.

**2.** Composé selon la revendication 1, où $R^1$ et $R^2$ sont les mêmes.

**3.** Composé selon l'une quelconque des revendications 1 et 2, où $R^1$ et $R^2$ sont choisis parmi $O(CH_2)_nC_mF_{2m+1}$ ou $NR^5S(O)_2(CH_2)_pC_mF_{2m+1}$ où $R_5$, p, m et n sont tels que définis à la revendication 1.

**4.** Composé selon l'une quelconque des revendications précédentes, où n et p sont indépendamment des entiers de 1 à 3 et m est un entier de 6 à 10.

**5.** Composé selon l'un quelconque des revendications précédentes, où $R^3$ est un groupe de formule $-(CH_2)_qOC(O)C(R^6)=CR^7R^8$ où q est un entier de 1 à 12 et $R^6$, $R^7$ et $R^8$sont indépendamment choisis parmi l'hydrogène ou d'un groupe $C_{1-4}$alkyle.

**6.** Composé selon la revendication 5, où $R^6$, $R^7$ et $R^8$ sont des atomes d'hydrogène.

**7.** Méthode de préparation d'un composé de formule (I) selon la revendication 1, ladite méthode comprenant la réaction d'un composé de formule (II)

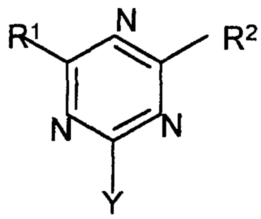

(II)

où $R^1$ et $R^2$ sont tels que définis à la revendication 1 et Y est un groupe partant, avec un composé de formule (III)

$$R^a\text{-X-}R^{3'} \quad (III)$$

où X est tel que défini à la revendication 1 et $R^{3'}$ est un groupe $R^3$ tel que défini à la revendication 1 ou un groupe précurseur qui peut être mis en réaction pour former un groupe $R^3$, et $R^a$ est un atome d'hydrogène ou un groupe $C_{1-3}$ alkyle ; et ensuite si nécessaire en convertissant un groupe $R^{3'}$ en un groupe $R^3$.

**8.** Méthode selon la revendication 7, où $R^{3'}$ est un groupe de formule $(CH_2)_qOH$ et est ensuite converti en $R^3$ par réaction avec un halogénure d'acide de formule $ZC(O)C(R^6)=CR^7R^8$ où Z est un halogène et $R^6$, $R^7$ et $R^8$ sont définis selon la revendication 5 en présence d'une base.

**9.** Méthode selon l'une quelconque des revendications 7 et 8, où le composé de formule (II) est préparé en faisant réagir un composé de formule (IV)

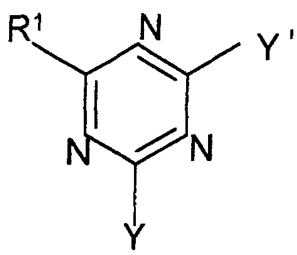

(IV)

où $R^1$ est défini selon la formule (I), Y est défini selon la formule (II) et Y' est un groupe partant, avec un composé de formule (V)

$$R^2H \quad (V)$$

où $R^2$ est défini selon la formule (I) en présence d'une base.

**10.** Méthode selon la revendication 9, où le composé de formule (IV) est préparé en faisant réagir un composé de formule (VI)

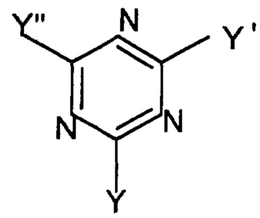

(VI)

où Y, Y' et Y" sont les mêmes ou différents groupes partants,
avec un composé de formule (VII)

$$R^1 H \qquad (VII)$$

où R' est tel que défini à la revendication 1 en présence d'une base.

**11.** Méthode selon la revendication 10, dans laquelle le composé de formule (IV) est converti en un composé de formule (II) in situ.

**12.** Composé polymérique qui a été dérivé à partir d'un composé de formule (I) selon la revendication 1.

**13.** Composé polymérique selon la revendication 12, qui comprend un polymère ou un co-polymère comprenant des unités répétées de formule (VIII)

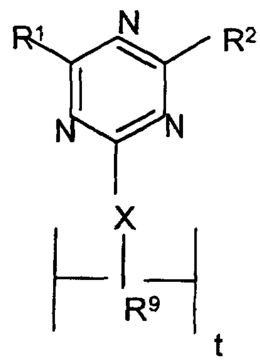

(VIII)

où $R^1$, $R^2$ et X sont définis selon la formule (I), t est un entier en excès de 5, et $R^9$ est un dérivé saturé de $R^3$ tel que défini selon la formule (I).

**14.** Composé polymérique selon la revendication 13, où $XR^9$ est un groupe fonctionnel de formule (IX)

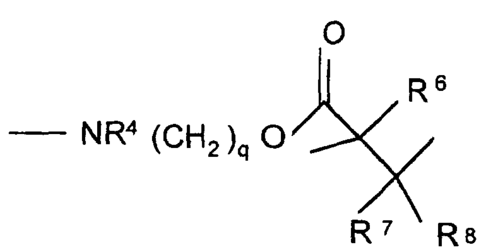

(IX)

où q, $R^6$, $R^7$ et $R^8$ sont tels que définis à la revendication 5.

**15.** Substrat qui est recouvert d'un composé polymérique selon l'une quelconque des revendications 12 à 14.

**16.** Substrat selon la revendication 15 qui est un tissu.